# EUROPEAN PATENT APPLICATION

(11) **EP 4 678 718 A1**
(43) Date of publication of application: **14.01.2026**
(21) Application number: 24767024.3
(22) Date of filing: 29.02.2024
(51) Int. Cl.: C10G 2/00, B01J 29/072, C10K 1/00

(54) **METHOD AND DEVICE FOR SUPPLYING TO CATALYST**

(30) Priority: 08.03.2023 JP 2023035210
(71) Applicant: JFE Engineering Corporation, Chiyoda-ku, Tokyo 100-0011 (JP); National University Corporation University of Toyama, Toyama-shi, Toyama 930-8555 (JP)
(72) Inventor: DOI, Makoto, Tokyo 100-0011 (JP); TSUBAKI, Noritatsu, Toyama-shi, Toyama 930-8555 (JP); GUO, Xiaoyu, Toyama-shi, Toyama 930-8555 (JP)
(74) Representative: Hoffmann Eitle
(86) International application number: PCT/JP2024/007603
(87) International publication number: WO 2024/185654

(57) **Abstract**

An object is to suppress deterioration in a catalyst capable of synthesizing a hydrocarbon from a synthesis gas containing carbon oxide. A supply method for supplying a synthesis gas containing hydrogen gas and carbon oxide gas to a catalyst capable of producing a hydrocarbon from the synthesis gas includes supplying the synthesis gas to the catalyst as a gas containing a poisoning substance that is poisonous to the catalyst. The concentration of the poisoning substance in the synthesis gas is set to more than 0 volppm and 1.0 volppm or less, preferably 0.01 volppm or more and 0.2 volppm or less, and more preferably 0.02 volppm or more and less than 0.1 volppm. The poisoning substance is a temporary poisoning substance which is at least one compound selected from the group consisting of NH₃, HCN, PH₃, NaCl, and KCl, or a permanent poisoning substance which is a compound including at least one of a compound containing S and HCl and is at least one compound selected from the group consisting of H₂S, COS, HCl, and AsH₃.

## Description

### Field

The present invention relates to a method and device for supplying to a catalyst, and is particularly suitably applied to a catalyst for producing a liquid fuel by reacting a mixed gas of carbon oxide and hydrogen.

### Background

In recent years, research and development for improving selectivity of a target product by controlling a complicated reaction path using a synthesis technique according to a Fischer-Tropsch (FT) method for synthesizing a liquid hydrocarbon from carbon monoxide (CO) and hydrogen (H₂) have been actively conducted.

Patent Literature 1 discloses a hydrocarbon production method including a first reaction step of supplying a gas containing carbon monoxide and hydrogen to a first reactor including an FT catalyst for producing a hydrocarbon by a reaction according to an FT method to produce a hydrocarbon, a second reaction step of supplying a gas to a second reactor including an FT catalyst for producing a hydrocarbon by a reaction by an FT method to produce a hydrocarbon, a first regeneration step of stopping the supply of the gas to the first reactor and regenerating the performance of the FT catalyst included in the first reactor, and a second regeneration step of stopping the supply of the gas to the second reactor and regenerating the performance of the FT catalyst included in the second reactor, the method performing the first regeneration step and the second regeneration step in mutually different periods. Patent Literature 2 discloses a catalyst regeneration method of raising the temperature of a catalyst to remove wax and then lowering the temperature to flow an oxidizing gas or a reducing gas for regeneration.

Non Patent Literature 1 discloses a regeneration treatment method after poisoning of an FT catalyst, which generates a hydrocarbon by a reaction according to the FT method, lowers CO conversion, that is, the activity of the catalyst, resulting in poisoning deterioration. Non Patent Literature 1 discloses a regeneration treatment method of washing a heptane solvent at a temperature of 100°C to remove excess wax, heating the heptane solvent in a heating furnace such as a fluidized bed using a mixed gas of air and nitrogen (N₂) to perform an oxidation treatment, gradually increasing the oxygen concentration to 3 to 21%, and performing reduction with hydrogen in a fluidized bed unit.

### Citation List

### Patent Literature

Patent Literature 1: JP 2022-102703 A
Patent Literature 2: JP 2022-535946 A

### Non Patent Literature

Non Patent Literature 1: "Fundamental understanding of deactivation and regeneration of cobalt Fischer-Tropsch synthesis catalysts", A.M. Saiba et al., Catalysis Today 154 (2010) 271-282

### Summary

### Technical Problem

As described above, in a catalyst capable of synthesizing a hydrocarbon from a synthesis gas containing carbon oxide, the activity decreases with time due to coating of the surface of the catalyst with the hydrocarbon or coking. Therefore, it is necessary to perform a regeneration treatment in which a synthesis reaction is stopped after the synthesis reaction is performed for a certain period of time to remove combustion or reaction by oxygen or hydrogen-containing gas. However, the regeneration treatment requires a long time of about several days to one week including the shutdown and the start-up of apparatus, and the synthesis of hydrocarbons cannot be performed during execution of the regeneration treatment, resulting in low production and high cost due to the production of oxygen gas and hydrogen gas required for the treatment, and therefore, a technique for suppressing a decrease in the activity of the catalyst, that is, deterioration of the catalyst, has been required.

The present invention has been made in view of the above, and an object thereof is to provide a method and device for supplying to a catalyst capable of suppressing deterioration in a catalyst capable of synthesizing a hydrocarbon from a synthesis gas containing carbon oxide gas.

### Solution to Problem

(1) To resolve the above problem and attain the object, according to an embodiment of the present invention, a method for supplying a synthesis gas containing hydrogen gas and carbon oxide gas to a catalyst capable of producing a hydrocarbon from the synthesis gas, the method includes: supplying the synthesis gas to the catalyst as a gas containing a poisoning substance that is poisonous to the catalyst.
(2) In the method for supplying a synthesis gas to a catalyst according to an embodiment of the present invention, a concentration of the poisoning substance in the synthesis gas is more than 0 volppm and 1.0 volppm or less in the above invention (1).
(3) In the method for supplying a synthesis gas to a catalyst according to an embodiment of the present invention, a concentration of the poisoning substance in the synthesis gas is 0.01 volppm or more and 0.2 volppm or less in the above invention (1).
(4) In the method for supplying a synthesis gas to a catalyst according to an embodiment of the present invention, a concentration of the poisoning substance in the synthesis gas is 0.02 volppm or more and less than 0.1 volppm in the above invention (1).
(5) In the method for supplying a synthesis gas to a catalyst according to an embodiment of the present invention, the poisoning substance is a temporary poisoning substance having a temporary poisonous property capable of regenerating the catalyst in any of the above inventions (1) to (4).
(6) In the method for supplying a synthesis gas to a catalyst according to an embodiment of the present invention, the temporary poisoning substance is at least one compound selected from the group consisting of ammonia (NH₃), hydrogen cyanide (HCN), phosphine (PH₃), sodium chloride (NaCl), and potassium chloride (KCl) in the above invention (5).
(7) In the method for supplying a synthesis gas to a catalyst according to an embodiment of the present invention, the poisoning substance is a permanent poisoning substance having a permanent poisonous property incapable of regenerating the catalyst in any of the above inventions (1) to (4).
(8) In the method for supplying a synthesis gas to a catalyst according to an embodiment of the present invention, the permanent poisoning substance is at least one compound selected from a compound containing sulfur (S) and hydrogen chloride (HCl) in the above invention (7).
(9) In the method for supplying a synthesis gas to a catalyst according to an embodiment of the present invention, the permanent poisoning substance is at least one compound selected from the group consisting of hydrogen sulfide (H₂S), carbonyl sulfide (COS), hydrogen chloride (HCl), and arsine (AsH₃) in the above invention (7) or (8).
(10) In the method for supplying a synthesis gas to a catalyst according to an embodiment of the present invention, in any of the above inventions (1) to (9), the catalyst includes a metal-based catalyst that contains a metal compound having activity in a Fischer-Tropsch synthesis reaction and produces a hydrocarbon from the synthesis gas, and a carrier catalyst that contains a metal oxide supporting the metal-based catalyst.
(11) In the method for supplying a synthesis gas to a catalyst according to an embodiment of the present invention, in any of the above inventions (1) to (10), the catalyst includes a metal-based catalyst that contains a metal and a metal compound having activity in a Fischer-Tropsch synthesis reaction and produces a hydrocarbon from the synthesis gas, and a carrier catalyst that contains a zeolite supporting the metal-based catalyst, the metal and the metal compound including cobalt and at least one metal selected from the group consisting of manganese and ruthenium.
(12) The method for supplying a synthesis gas to a catalyst according to an embodiment of the present invention, includes controlling, by a control unit including hardware, an amount of the poisoning substance introduced into the synthesis gas supplied to the catalyst to adjust a concentration of the poisoning substance contained in the synthesis gas supplied to the catalyst in any of the above inventions (1) to (11).
(13) In the method for supplying a synthesis gas to a catalyst according to an embodiment of the present invention, before supplying the synthesis gas to the catalyst, the control unit branches at least a part of the synthesis gas from a line through which the synthesis gas is supplied to the catalyst, removes the poisoning substance at a preceding stage of the catalyst, and then supplies the synthesis gas to the catalyst together with a remainder of the synthesis gas in the above invention (12).
(14) The method for supplying a synthesis gas to a catalyst according to an embodiment of the present invention, includes recovering the poisoning substance on a discharge side of the catalyst; and mixing at least a part of the recovered poisoning substance into the synthesis gas supplied to the catalyst in the above invention (12) or (13) .
(15) A supply device according to an embodiment of the present invention, includes: a synthesis gas supply unit configured to supply a synthesis gas containing hydrogen gas, carbon oxide gas, and a poisoning substance that is poisonous to a catalyst; and a gas purification unit configured to adjust a concentration of the poisoning substance contained in the synthesis gas supplied from the synthesis gas supply unit. Further, the supply device is configured to supply the synthesis gas to a synthesis reactor including the catalyst, the synthesis reactor being configured to synthesize a hydrocarbon from the hydrogen gas and the carbon oxide gas contained in the synthesis gas.
(16) In the supply device according to an embodiment of the present invention, the gas purification unit adjusts the concentration of the poisoning substance in the synthesis gas to be more than 0 volppm and 1.0 volppm or less in the above invention (15).
(17) In the supply device according to an embodiment of the present invention, the gas purification unit includes at least one poisoning substance separation unit configured to separate the poisoning substance from the synthesis gas, and a control unit including hardware capable of controlling a flow rate of supplying the synthesis gas to the poisoning substance separation unit, at a preceding stage of the poisoning substance separation unit, the synthesis gas is caused to flow into at least one of a first route in which the synthesis gas is supplied to the synthesis reactor without passing through the poisoning substance separation unit and a second route in which the synthesis gas is supplied to the synthesis reactor after being supplied to the poisoning substance separation unit, and the control unit adjusts the concentration of the poisoning substance by controlling the flow rate of the synthesis gas flowing into the first route and the flow rate of the synthesis gas flowing into the second route in the above invention (15) or (16).
(18) The supply device according to an embodiment of the present invention, includes a plurality of stages of the poisoning substance separation unit in series along a flow direction of the synthesis gas in any of the inventions (15) to (17). Further, the supply device is configured to adjust the concentration of the poisoning substance by selecting the number of stages of the poisoning substance separation unit to which the synthesis gas is supplied.
(19) A supply device according to an embodiment of the present invention, includes a synthesis gas supply unit configured to supply a synthesis gas containing hydrogen gas and carbon oxide gas; and a poisoning substance addition unit capable of adding a poisoning substance that is poisonous to a catalyst to the synthesis gas supplied from the synthesis gas supply unit. Further, the supply device is configured to supply the synthesis gas to a synthesis reactor including the catalyst, the synthesis reactor being configured to synthesize a hydrocarbon from the hydrogen gas and the carbon oxide gas contained in the synthesis gas.
(20) In the supply device according to an embodiment of the present invention, the poisoning substance addition unit adds the poisoning substance to the synthesis gas so that a concentration of the poisoning substance in the synthesis gas is more than 0 volppm and 1.0 volppm or less in the above invention (19).
(21) The supply device according to an embodiment of the present invention, includes a plurality of poisoning substance separation units configured to recover and release the poisoning substance in the above invention (19) or (20). Further, on a downstream side of the synthesis reactor along a flow direction of the synthesis gas, some of the poisoning substance separation units among the plurality of poisoning substance separation units are configured to recover the poisoning substance, and on an upstream side of the synthesis reactor, the remaining poisoning substance separation units of the plurality of poisoning substance separation units are configured to introduce the poisoning substance into the synthesis gas.
(22) In the supply device according to an embodiment of the present invention, the some of the poisoning substance separation units and the remaining poisoning substance separation units are configured to be exchangeable in the above invention (21).
(23) In the supply device according to an embodiment of the present invention, in any one of the above inventions (15) to (22), the catalyst includes a metal-based catalyst that contains a metal and a metal compound having activity in a Fischer-Tropsch synthesis reaction and produces a hydrocarbon from the synthesis gas, and a carrier catalyst that contains a zeolite supporting the metal-based catalyst, the metal and the metal compound including cobalt and at least one metal selected from the group consisting of manganese and ruthenium.

### Advantageous Effects of Invention

According to the method and device for supplying to a catalyst according to the present invention, it is possible to suppress deterioration in a catalyst capable of synthesizing a hydrocarbon from a synthesis gas containing carbon oxide.

### Brief Description of Drawings

FIG. 1 is a graph showing an example of CO conversion over time with a catalyst used in a synthesis reaction according to an embodiment of the present invention and the prior art.
FIG. 2 is a graph showing pore size distribution of various samples of an FT synthesis catalyst according to an embodiment of the present invention.
FIG. 3 is a graph showing the poisoning substance concentration dependence of the rate of decline in reactivity of a FT synthesis catalyst according to an embodiment of the present invention.
FIG. 4 is a schematic view illustrating a method for supplying a synthesis gas to a catalyst according to a first example in an embodiment of the present invention.
FIG. 5 is a schematic view illustrating a specific example of a synthesis gas supply unit including a poisoning substance according to a first example in an embodiment of the present invention.
FIG. 6 is a block diagram illustrating a gas purifier according to a first example in an embodiment of the present invention.
FIG. 7 is a schematic view illustrating a method for supplying a synthesis gas to a catalyst according to a second example in an embodiment of the present invention.
FIG. 8 is a schematic view illustrating Example 1 of a synthesis gas supply unit according to a second example in an embodiment of the present invention.
FIG. 9 is a block diagram illustrating a gas purifier according to a second example in an embodiment of the present invention.

### Description of Embodiments

Hereinafter, embodiments of the present invention will be described with reference to the drawings. In addition, the present invention is not limited by the embodiments described below. First, as an embodiment of the present invention, experiments and intensive studies conducted by the present inventors to achieve the above-described object will be described.

First, the present inventors used an FT (Fischer-Tropsch) synthesis catalyst as a catalyst capable of synthesizing a hydrocarbon from a synthesis gas containing hydrogen gas and carbon oxide gas. The FT synthesis catalyst is a catalyst configured to include a metal-based catalyst that contains a metal and a metal compound, which have activity in a Fischer-Tropsch (FT) synthesis reaction and produces a hydrocarbon from a synthesis gas, and a carrier catalyst that contains zeolite supporting the metal-based catalyst. Examples of the FT synthesis catalyst include a catalyst in which a metal catalyst is supported on pores subjected to cation exchange treatment using Y-type pore zeolite as a carrier catalyst. As the carrier catalyst, various metal oxides such as alumina (Al₂O₃) and silica (SiO₂) can be adopted in addition to zeolite.

In addition, the FT synthesis catalyst includes, for example, a metal-based catalyst that contains a metal and a metal compound having activity in the FT synthesis reaction and produces a hydrocarbon from a synthesis gas, and a carrier catalyst that contains zeolite supporting the metal-based catalyst, and is a catalyst capable of producing a hydrocarbon from the synthesis gas.

Here, the metal and the metal compound having activity in the FT synthesis reaction preferably contain cobalt (Co) and at least one metal selected from the group consisting of manganese (Mn) and ruthenium (Ru), preferably two metals. Here, the supported amount of Mn is preferably 1 wt% or more and 3 wt% or less, the supported amount of Ru is preferably 0.5 wt% or more and 2 wt% or less, and the supported amount of Co is preferably 10 wt% or more and 30 wt% or less.

Furthermore, zeolite carrying the metal-based catalyst preferably includes zeolite having pores that decompose carbon chains of the generated hydrocarbon, and the pores are mesopores having an opening diameter of 2 nm or more and 50 nm or less. The ratio of silicon to aluminum (Si/Al ratio) in the zeolite is preferably 2.5 or more and 3.5 or less.

The production of the catalyst as described above includes a pore forming step of forming mesopores in a carrier catalyst and a catalyst supporting step of supporting a metal and a metal compound on the carrier catalyst. Then, a liquid fuel composed of a hydrocarbon is produced from a synthesis gas by a Fischer-Tropsch synthesis reaction.

Here, as Example 1 of the catalyst supporting step, a method including a step of causing a carrier catalyst to support a metal and a metal compound containing Co, and at least one of a metal and a metal compound containing Mn and a metal and a metal compound containing Ru, preferably both of them can be adopted. In this case, the catalyst supporting step preferably includes a melt impregnation step of melt-impregnating the carrier catalyst with the metal compound containing Co and at least one, preferably both, of the metal compound containing Mn and the metal compound containing Ru. Here, the melt impregnation step is a step of supporting the metal compound containing Co on the carrier catalyst by the melt impregnation method, and then supporting at least one of the metal compound containing Mn and the metal compound containing Ru by the melt impregnation method. Alternatively, the melt impregnation step is a step of substantially simultaneously supporting the metal compound containing Co and at least one of the metal compound containing Mn and the metal compound containing Ru on the carrier catalyst by a melt impregnation method.

In addition, a method including an impregnation step of supporting a metal compound containing Co on a carrier catalyst by an impregnation method, then immersing the carrier catalyst on which Co is supported in a solution containing at least one of a solution containing Mn and a solution containing Ru or a solution containing both to impregnate the carrier catalyst and the supported catalyst supported on the carrier catalyst can be adopted as Example 2 of the catalyst supporting step.

Furthermore, a method including an impregnation step of supporting a metal compound containing Co on a carrier catalyst by an impregnation method and also immersing the carrier catalyst in a solution of at least one of a solution containing Mn and a solution containing Ru or both solutions to impregnate at least one of the carrier catalyst and the supported catalyst supported on the carrier catalyst can be adopted as Example 3 of the catalyst supporting step.

Moreover, a method including a melt impregnation step of melt-impregnating a carrier catalyst with a metal compound containing Co, and an impregnation step of immersing the carrier catalyst on which the metal compound containing Co is supported in at least one of a solution containing Mn and a solution containing Ru or both solutions to impregnate at least one of the carrier catalyst and the supported catalyst supported on the carrier catalyst in the melt impregnation step can be adopted as Example 4 of the catalyst supporting step.

As the carrier catalyst, it is preferable to adopt a carrier catalyst in which a cation is ligated in advance or a cation is ligated by a cation exchange step using an ion exchange method executed before the catalyst supporting step. Here, the cation is preferably, but not limited to, at least one cation selected from the group consisting of lanthanum, potassium, lithium, sodium, and cerium.

In the reaction for generating a hydrocarbon by the FT method, carbon (C) is produced from carbon oxide such as carbon monoxide (CO) or carbon dioxide (CO₂) on the surface of the FT synthesis catalyst, and a hydrocarbon is synthesized by a synthesis reaction with hydrogen. In this synthesis reaction, carbon is deposited (carbon deposition) on the surface of the catalyst over time, so-called coking occurs, or a high-melting-point hydrocarbon (wax) adheres to the catalyst, deteriorating the catalyst and lowering the activity.

The present inventors conducted various experiments and studies in order to delay the state in which the activity decreases. First, the present inventors conceived a method of adsorbing a material (hereinafter, referred to as a poisoning substance or a catalyst poisoning substance) that was poisonous to a catalyst having strong adsorptivity to the surface of the catalyst in advance. As a result, on the surface of the catalyst, the poisoning substance is adsorbed first to a portion serving as a starting point of the occurrence of coking (carbon deposition) and the coating of the high-melting-point hydrocarbon, and therefore, the occurrence and growth of coking can be suppressed. That is, the present inventors conceived that in a synthesis reaction for synthesizing a hydrocarbon from carbon oxide and hydrogen using a catalyst, it was preferable to add a small amount of a poisoning substance to a synthesis gas supplied to the catalyst or to supply a synthesis gas containing the poisoning substance to the catalyst in advance.

The present inventors conducted a verification experiment based on the above studies. That is, the present inventors measured a change in CO conversion (%) over time when a synthesis gas containing hydrogen sulfide (H₂S) as a poisoning substance at a concentration of 0.1 volppm was introduced into an FT synthesis reactor containing an FT synthesis catalyst. Note that components in the synthesis gas other than impurity components are H₂ and CO, and in the present embodiment, the ratio of H₂ to CO (H₂/CO) is preferably two or more (H₂/CO ≥ 2). Here, the concentration ratio is H₂:CO = 2:1.

That is, a synthesis experiment (hereinafter, referred to as a poisoning synthesis test) using a synthesis gas containing a poisoning substance was performed by an FT method. Furthermore, the present inventors subsequently measured the change in CO conversion (%) over time when a synthesis gas containing ammonia (NH₃) as a poisoning substance at a concentration of 0.1 volppm was introduced into the FT synthesis reactor. Thereafter, when the CO conversion decreased to about 98%, reduction treatment with hydrogen (H₂) was performed in the same manner as in the prior art. In addition, as a comparison, the present inventors conducted a synthesis experiment (synthesis test) according to a conventional FT method using a synthesis gas containing no poisoning substance, and measured the change in CO conversion (%) over time.

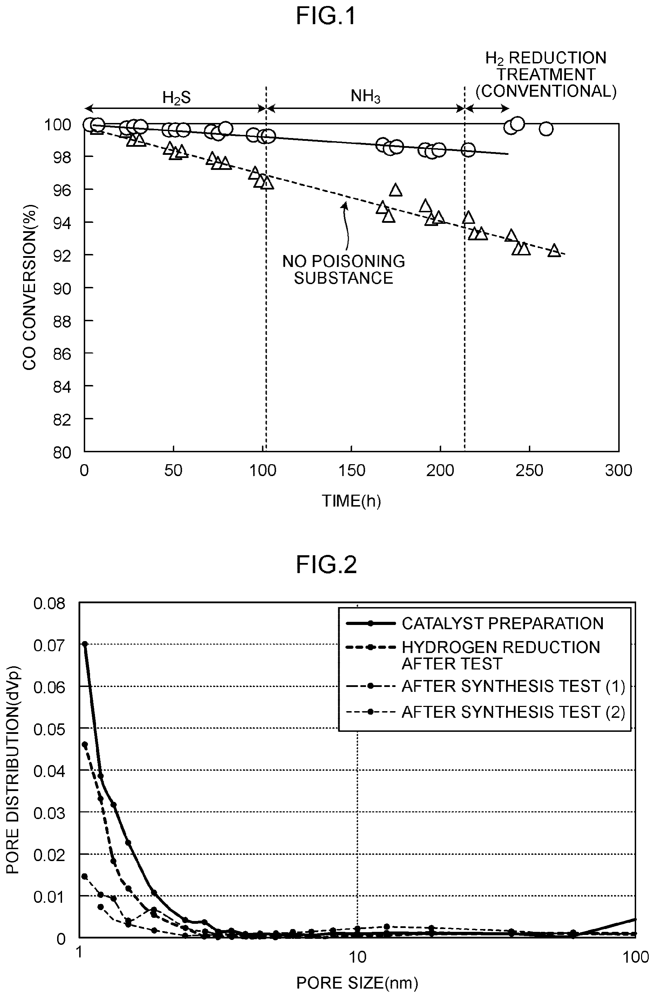

FIG. 1 is a graph showing results of measuring CO conversion over time when a poisoning substance is included in a synthesis gas and when the poisoning substance is not included in the synthesis gas in a poisoning synthesis test. Table 1 below shows the poisoning substance contained in the synthesis gas, the concentration, and the decline rate of CO conversion (hereinafter, referred to as the rate of decline in CO conversion (%/h)) in the synthesis gas containing no poisoning substance, which were derived from the results shown in FIG. 1. In addition, Table 2 shows conditions of the temperature of the catalyst and the average temperature of the reaction tube including the catalyst in the synthesis reaction in the experiment, and the CO conversion when the catalyst is regenerated.

**Table 1**

| Poisoning substance | Concentration (volppm) | Rate of decline in CO conversion (%/h) |
|---|---|---|
| H₂S | 0.1 | 0.0048 |
| NH₃ | 0.1 | 0.0082 |
| None | 0 | 0.0287 |

**Table 2**

| Item | Catalyst average temperature (°C) | Catalyst & reaction tube average temperature (°C) | CO conversion ratio(%) |
|---|---|---|---|
| Poisoning test | 261.6 | 257.0 | 99.8 |
| Synthesis test after hydrogen reduction | 261.8 | 257.0 | 99.8 |

From FIG. 1 and Table 1, it can be seen that the rate of decline in CO conversion is 0.0287 (%/h) when the synthesis gas containing no poisoning substance according to the prior art is introduced into the FT synthesis reactor containing the FT synthesis catalyst. When a synthesis gas containing 0.1 volppm of hydrogen sulfide (H₂S) is introduced into the FT synthesis reactor, the rate of decline in CO conversion is 0.0048 (%/h), and it is found that the CO conversion decreases to about (0.0048/0.0287 ≈) 1/5 as compared with the conventional case. Similarly, when a synthesis gas containing 0.1 volppm of ammonia (NH₃)is introduced into the FT synthesis reactor, it is found that the rate of decline in CO conversion is 0.0082 (%/h), which decreases to about (0.0082/0.0287 ≈) 1/3 as compared with the conventional case. That is, it is found that when the synthesis gas contains H₂S or NH₃ as a poisoning substance, the rate of decline in CO conversion (%/h) decreases to about 1/5 to 1/3, and deterioration is thus delayed.

From Table **2,** it can be seen that the catalyst temperature and the CO conversion at the start of the poisoning test are equal to the catalyst temperature and the CO conversion when the synthesis test is conducted again after the catalyst is regenerated by hydrogen reduction after the poisoning test. That is, it can be seen from Table 2 that the catalyst is sufficiently regenerable.

Furthermore, the present inventors also studied deterioration factors. That is, the total carbon concentration and the hydrocarbon analytical concentration were measured when the synthesis test using the synthesis gas containing no poisoning substance was conducted for 100 hours and when the poisoning synthesis test using the synthesis gas containing 1 volppm of the poisoning substance was conducted for 100 hours. Note that the total carbon concentration of the catalyst in the adjusted state before the synthesis test was also measured. Here, the total carbon concentration was measured by a combustion-infrared absorption method, and the hydrocarbon-based analytical concentration was measured by temperature programmed desorption-mass spectrometry (TPD-MS method). Table 3 shows the results.

**Table 3**

| | Total carbon concentration (combustion and infrared absorption method) () indicating catalytic conversion | Hydrocarbon-based analytical concentration (TPD-MS method) |
|---|---|---|
| As catalyst preparation | 0.02% (0.1%) | - |
| After 100 hour synthesis test (S = 0 volppm) | 6.56% (32.8%) | 3.6 volppm (tetradecene, undecene) |
| After 100 hour synthesis test (S = 1 volppm) | 0.42% (2.1%) | 1.1 volppm (tetradecene, undecene) |

From Table 3, as a result of a synthesis test using a synthesis gas containing no sulfur (S) as a poisoning substance, the total carbon concentration was 6.56%, and the catalyst ratio was 32.8%. On the other hand, as a result of a poisoning synthesis test using a synthesis gas containing S as a poisoning substance, it was confirmed that the total carbon concentration was 0.42%, the catalyst ratio was 2.1%, and the total carbon concentration was reduced to about (0.42/6.56 ≈) 1/15. In addition, from the measurement results of the hydrocarbon-based analytical concentration by temperature programmed desorption-mass spectrometry (TPD-MS), it is considered that the wax adhered is heated by heating at constant temperature rise, and gasified and desorbed by pyrolysis formation, and detected as tetradecene or undecene. From the gas concentration ratio, it can be estimated that when the synthesis gas contains the poisoning substance, the wax adhesion rate is reduced to (1.1/3.6 ≈) 1/3 or less as compared with the case where the synthesis gas does not contain the poisoning substance.

Here, according to the studies by the present inventors, the wax adhering to the catalyst is presumed to be an aliphatic hydrocarbon-based compound such as paraffin, and the reason why tetradecene and undecene are less than the total number of carbon atoms is presumed to be that a high-boiling point wax that is not thermally decomposed by heating at constant temperature rise still remains. As described above, regarding deterioration factors of the catalyst, the catalyst analysis after the synthesis test revealed that the adhered material was waxes, and it was confirmed that the adhered amount of waxes and the like was reduced in the case of synthesis gas containing the poisoning substance. In addition, when a Co-supported pore zeolite catalyst is used as the FT synthesis catalyst, coking and the volume of wax are likely to occur in highly active pores. Therefore, the above is considered to be more effective when the Co-supported pore zeolite catalyst is used as the FT synthesis catalyst.

Furthermore, the present inventors measured the pore size distribution and the specific surface area in the zeolite carrier of the FT synthesis catalyst. As a sample, an FT synthesis catalyst in a state in which the catalyst was prepared (catalyst preparation), a state after the synthesis tests (1) and (2), and a state after the hydrogen reduction treatment following the test (hydrogen reduction after the test) was used. FIG. 2 is a graph showing a pore size distribution in a sample including the above-described FT synthesis catalyst. Table 4 shows results of deriving the specific surface area based on the N₂ adsorption isotherm results in the sample composed of the above-described FT synthesis catalyst.

The synthesis test (1), for example, is conducted under conditions where the ratio of H₂ to CO in the synthesis gas is 1.5 times, the pressure is 2 MPa, the catalyst temperature is constant at 250°C (at the gas upstream end), and no poisoning substances are contained for 260 hours continuously. The synthesis test (2), for example, is conducted under conditions where the ratio of H₂ to CO in the synthesis gas is doubled, the pressure is 2 MPa, the catalyst temperature is constant at 260°C (at the gas upstream end), and no poisoning substances are contained for 260 hours continuously.

**Table 4**

| Sample | Specific surface area (m²/g) |
|---|---|
| Catalyst preparation | 562 |
| After synthesis test (1) | 24 |
| After synthesis test (2) | 76 |
| Hydrogen reduction after test | 263 |

From Table 4, it can be seen that in the nitrogen (N₂) adsorption isotherm results, the specific surface area of the FT synthesis catalyst after the synthesis tests (1) and (2) is significantly reduced. That is, it is found that the specific surface area of the FT synthesis catalyst is 562 m²/g before the synthesis test, but is reduced to about 1/23 to 24 m²/g after the synthesis test (1), and is reduced to about 1/7 to 76 m²/g after the synthesis test (2). In addition, it can be seen from FIG. 2 that the specific surface area of pores having a pore size of about 1 nm to 5 nm is significantly reduced after the synthesis tests (1) and (2) also in the pore distribution analysis. According to the studies by the present inventors based on these results, it is considered that the cause is that pores of the zeolite carrier of the FT synthesis catalyst are blocked with waxes.

Next, the present inventors conducted experiments and studies on the dependence of the rate of decline in reactivity (%/h) on the poisoning substance concentration based on the above studies. That is, the present inventors variously changed the concentration of the poisoning substance contained in the synthesis gas and measured the rate of decline in reactivity (%/h). The poisoning substance used here include a poisoning substance (hereinafter, referred to as a temporary poisoning substance) that is regenerable by hydrogen reduction treatment even after being adsorbed on the FT synthesis catalyst and temporarily poisonous (temporary poisonous property) and a poisoning substance (hereinafter, referred to as a permanent poisoning substance) having permanent poisonous properties that poisons the FT synthesis catalyst in an unrenewable state.

The temporary poisoning substance is a substance composed of a reversible catalyst poison such as condensation or deposition by physical adsorption or the like by, for example, intermolecular force with respect to the FT synthesis catalyst, and specific examples thereof include ammonia (NH₃), hydrogen cyanide (HCN), phosphine (PH₃), sodium chloride (NaCl), and potassium chloride (KCl).

The permanent poisoning substance is a substance composed of catalyst poison irreversibly adsorbed to the FT synthesis catalyst, for example, chemically adsorbed, and examples thereof include a compound containing sulfur (S) and a compound containing at least one of hydrogen chloride (HCl), and specific examples thereof include hydrogen sulfide (H₂S), carbonyl sulfide (COS), hydrogen chloride (HCl), and arsine (AsH₃).

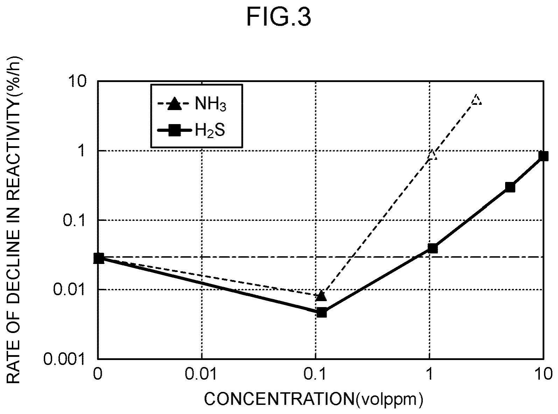

FIG. 3 is a graph showing the poisoning substance concentration dependence of the rate of a decline in reactivity of the FT synthesis catalyst according to the present embodiment. In the graph shown in FIG. 3, ammonia (NH₃)is used as an example of the temporary poisoning substance, and hydrogen sulfide (H₂S) is used as an example of the permanent poisoning substance. In the graph shown in FIG. 3, the concentration of 0 volppm indicates a conventional case where the synthesis gas supplied to the FT synthesis catalyst does not contain a poisoning substance, and the rate of decline in reactivity in the case where the synthesis gas does not contain a poisoning substance is indicated by an alternate long and short dash line.

It can be seed from FIG. 3 that, when the synthesis gas contains a permanent poisoning substance as a poisoning substance (in FIG. 3, a thick solid line), the rate of decline in reactivity in the case where the concentration of the poisoning substance is more than 0 and less than 1 volppm is equal to or less than the rate of decline in reactivity (in FIG. 3, an alternate long and short dash line) in the case where the synthesis gas does not contain the poisoning substance. Similarly, as can be seen from FIG. 3, when the synthesis gas contains a temporary poisoning substance as a poisoning substance (in FIG. 3, a dotted line ), the rate of decline in reactivity in the case where the concentration of the poisoning substance is more than 0 volppm and 0.2 volppm or less is equal to or less than the rate of decline in reactivity (in FIG. 3, an alternate long and short dash line) in the case where the synthesis gas does not contain the poisoning substance. It can be seen from FIG. 3 that the rate of decline in reactivity is minimized when the concentration is 0.1 volppm regardless of whether the poisoning substance is a temporary poisoning substance or a permanent poisoning substance. That is, as can be seen from FIG. 3, the rate of decline in reactivity when the concentration of the poisoning substance in the synthesis gas is less than 0.1 volppm is equal to or less than the rate of decline in reactivity in the case where the synthesis gas does not contain the poisoning substance, regardless of whether the poisoning substance is a temporary poisoning substance or a permanent poisoning substance. In addition, it is found that the rate of decline in reactivity can be maintained at 0.01%/h or less when the concentration is 0.02 volppm or more. This is particularly pronounced when the poisoning substance is a permanent poisoning substance. From the above studies, the synthesis gas containing the poisoning substance can be supplied to the FT synthesis catalyst to reduce the rate of decline in reactivity, thus making the time until the FT synthesis catalyst cannot be used or the regeneration treatment becomes necessary longer.

From the above, it can be seen that the concentration of the poisoning substance in the synthesis gas is preferably more than 0 volppm and less than 1 volppm, more preferably more than 0 volppm and 0.2 volppm or less, and still more preferably 0.02 volppm or more and less than 0.1 volppm. When the concentration of the poisoning substance in the synthesis gas is about 0.1 volppm, it is preferable to set the concentration to, for example, 0.05 volppm or more and 0.15 volppm or less. When the synthesis gas contains a plurality of poisoning substances, the concentration of the poisoning substances is preferably the total concentration of the plurality of poisoning substances in consideration of a phenomenon of adsorption of the poisoning substances to the FT synthesis catalyst. When the concentration of the poisoning substance is more than 0 volppm, it may be 0.001 volppm or more, 0.01 volppm or more, or the like.

### (First Example)

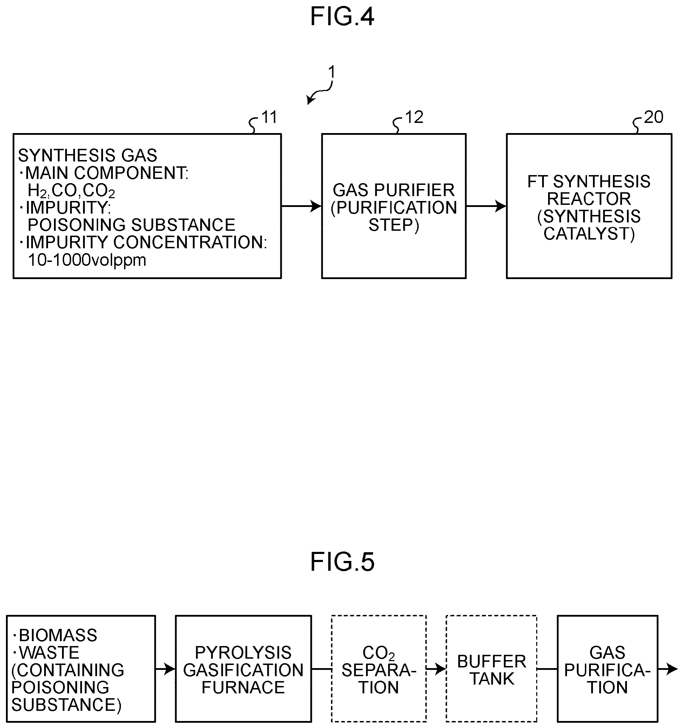

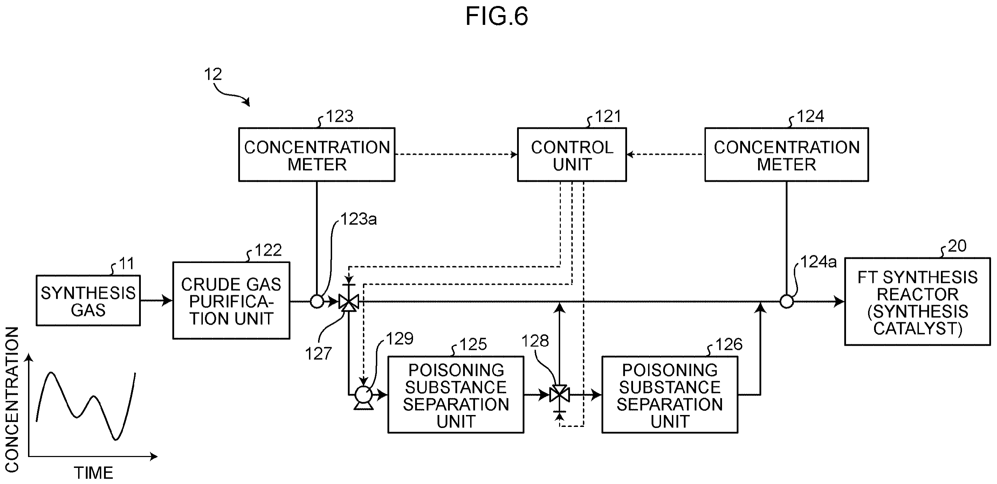

Next, examples based on the above studies by the present inventors will be described. FIG. 4 is a schematic view for describing a method for supplying a synthesis gas according to the first example. FIG. 5 is a schematic view illustrating a specific example of a synthesis gas supply unit 11 including a poisoning substance according to the first example. FIG. 6 is a block diagram illustrating a gas purifier 12 according to a first example.

As illustrated in FIG. 4, the synthesis gas contains a main component and impurities. Here, examples of the main component of the synthesis gas include hydrogen (H₂) and hydrocarbons such as carbon monoxide (CO) and carbon dioxide (CO₂). In addition, there is a possibility that various impurities are contained as impurities, and in the first example, a case where a temporary poisoning substance or a permanent poisoning substance is contained as a poisoning substance is considered.

That is, in the first example, the synthesis gas containing the poisoning substance is supplied from the synthesis gas supply unit 11 to the gas purifier 12. The synthesis gas supply unit 11 is configured to generate or store synthesis gas and supply the synthesis gas to the outside. The gas purifier 12 as a gas purification unit is configured to adjust the concentration of various gases contained in the synthesis gas. In the gas purifier 12 to which the synthesis gas containing the poisoning substance has been supplied, a gas purification step of adjusting the concentration of the poisoning substance contained in the synthesis gas to more than 0 volppm and 1 volppm or less, preferably 0.01 volppm or more and 0.1 volppm or less is performed. In this case, the concentration of the poisoning substance is preferably adjusted based on the concentration of the entire poisoning substance. In other words, it is preferable to adjust the concentration of each of a plurality of poisoning substances to set the total concentration of the plurality of poisoning substances to 0 volppm or more and 1 volppm or less.

Thereafter, the synthesis gas in which the concentration of the poisoning substance has been adjusted by the gas purifier 12 is supplied to the FT synthesis reactor 20, which contains the synthesis catalyst, to generate a hydrocarbon through the FT method. In the first example, the synthesis gas supply unit 11 and the gas purifier 12 constitute a synthesis gas supply device 1 as a supply device.

Here, as illustrated in FIG. 5, specifically, a method of thermally decomposing waste such as biomass and refuse with a gasification furnace or the like to generate H₂ gas and CO gas can be adopted as for the synthesis gas supply unit 11 including the poisoning substance according to the first example. Since CO₂ is also generated, it is also possible to install a buffer tank or the like corresponding to the separation of CO₂ and the fluctuation of gas. In FIG. 5, the dotted line portion may or may not be installed in the synthesis gas supply unit 11.

As illustrated in FIG. 6, a gas purifier 12 according to the first example includes a control unit 121, a crude gas purification unit 122, concentration meters 123 and 124, poisoning substance separation units 125 and 126, branch valves 127 and 128, and a pump 129.

Specifically, the control unit 121 includes a processor such as a central processing unit (CPU), a digital signal processor (DSP), or a field-programmable gate array (FPGA), and a main storage unit such as a random access memory (RAM) or a read only memory (ROM) (all not illustrated). Furthermore, the control unit 121 may include a storage unit (not illustrated) including a storage medium selected from an erasable programmable ROM (EPROM), a hard disk drive (HDD), a solid state drive (SSD), a removable medium, and the like. The removable medium is, for example, a universal serial bus (USB) memory, or a disk recording medium such as a compact disc (CD), a digital versatile disc (DVD), or a Blu-ray (registered trademark) disc (BD). An operating system (OS), various programs, various tables, various databases, and the like can be stored in the storage unit included in the control unit 121, and the stored program is loaded and executed in the work area of the main storage unit, and each constituent unit such as the branch valves 127 and 128 and the pump 129 is controlled through the execution of the program based on the poisoning substance concentration information supplied from the concentration meters 123 and 124, thus realizing a function meeting a predetermined purpose.

The crude gas purification unit 122 is configured to remove at least a part of, for example, NH₃, which is a temporary poisoning substance, and H₂S, which is a permanent poisoning substance, contained in the synthesis gas. The crude gas purification unit 122 includes, for example, a wet crude gas purifier, and is specifically configured to remove NH₃ contained in the synthesis gas and desulfurize H₂S, for example, by acid, alkali cleaning, or desulfurization, using a scrubber.

The concentration meters 123 and 124 include concentration sensors 123a and 124a that measure concentration, respectively. The concentration sensor 123a is configured to measure the concentration of the poisoning substance (poisoning substance concentration) in the synthesis gas on the discharge side of the crude gas purification unit 122. The concentration sensor 124a is configured to measure the concentration of the poisoning substance in the synthesis gas supplied to the FT synthesis reactor 20. Each of the poisoning substance separation units 125 and 126 includes, for example, an adsorption tower configured to adsorb a poisoning substance by activated carbon or the like.

According to the findings of the present inventors, the concentration of the poisoning substance in the synthesis gas in the synthesis gas supply unit 11 varies with the lapse of time as illustrated in the example of the graph in FIG. **6****.** Therefore, the present inventors conceived a method of measuring (monitoring) and controlling the concentration of the synthesis gas supplied to the gas purifier 12. According to the studies by the present inventors, the measurement of the synthesis gas is preferably performed at least for the concentration of the poisoning substance after being roughly purified and the concentration of the poisoning substance immediately before being supplied to the FT synthesis reactor. Furthermore, the present inventors conceived of controlling the concentration of the poisoning substance supplied to the FT synthesis reactor 20 to be about a predetermined value by branching the synthesis gas in at least two ways using a plurality of branch valves according to the concentration of the poisoning substance and adjusting the branch flow rate and the branch flow rate ratio thereof.

In the first example, the synthesis gas initially has an impurity concentration of about 10 to 1000 volppm. The synthesis gas is supplied from the synthesis gas supply unit 11 to the crude gas purification unit 122 while the impurity concentration and the poisoning substance concentration vary with time. The crude gas purification unit 122 adjusts the concentration of the poisoning substance in the synthesis gas to 0.1 volppm or more and 100 volppm or less. The concentration of the poisoning substance in the synthesis gas discharged from the crude gas purification unit 122 is measured the concentration sensor 123a. The measurement value measured by the concentration sensor 123a is output to the control unit 121 by the concentration meter 123.

The synthesis gas, from which the poisoning substance has been removed to adjust the concentration of the poisoning substance, is branched by the branch valve 127 while being flowed by the pump 129. The branch valve 127 is controlled by the control unit 121. The control unit 121 adjusts the branch flow rate of the synthesis gas by controlling the branch valve 127 and the pump 129, and performs adjustment to supply one branched synthesis gas as the first route to the FT synthesis reactor 20 without passing through the poisoning substance separation unit 125. The other branched synthesis gas is supplied to the poisoning substance separation unit 125 as the second route. In the poisoning substance separation unit 125, the poisoning substance contained in the flowed synthesis gas is separated and removed using, for example, activated carbon.

That is, when the control unit 121 determines that the concentration of the poisoning substance acquired from the concentration meter 123 is equal to or less than the predetermined value, the synthesis gas discharged from the crude gas purification unit 122 is directly passed through and supplied to the FT synthesis reactor 20.

When determining that the concentration of the poisoning substance acquired from the concentration meter 123 is higher than the predetermined value, the control unit 121 derives a necessary branch flow rate in the synthesis gas and controls the branch valve 127 to perform adjustment of supplying the synthesis gas to the poisoning substance separation unit 126 at the derived branch flow rate. In the poisoning substance separation unit 126, the poisoning substance contained in the flowed synthesis gas is separated and removed using, for example, activated carbon. A route passing through the poisoning substance separation unit 126 is also included in the second route.

The branch valve 128 is provided between the poisoning substance separation units 125 and 126, in other words, at the lower stage of the poisoning substance separation unit 125 and at the upper stage of the poisoning substance separation unit 126. The synthesis gas discharged from the poisoning substance separation unit 125 is branched by a branch valve 128. The branch valve 128 is controlled by the control unit 121. The control unit 121 controls the branch valve 128 to adjust the branch flow rate and the branch flow rate ratio of the synthesis gas. One synthesis gas branched by the branch valve 128 is supplied to the FT synthesis reactor 20. The other branched synthesis gas is further supplied to the poisoning substance separation unit 126.

The concentration of the poisoning substance in the synthesis gas supplied to the FT synthesis reactor 20 is measured by the concentration sensor 124a. The concentration of the poisoning substance of the synthesis gas supplied from the concentration meter 124 to the FT synthesis reactor 20 is input to the control unit 121. Here, when the concentration of the poisoning substance acquired from the concentration meter 124 is a predetermined value or less, for example, 0.1 volppm or less, the control unit 121 determines that the concentration of the poisoning substance is low. In this case, control is performed to increase the branch flow rate of the synthesis gas supplied to the FT synthesis reactor 20 without being supplied to the poisoning substance separation unit 125 among the synthesis gas discharged from the poisoning substance separation unit 126. When determining that the concentration of the poisoning substance acquired from the concentration meter 124 is low, the control unit 121 performs control to increase the branch flow rate of the synthesis gas supplied to the FT synthesis reactor 20 without passing through the poisoning substance separation unit 125 among the synthesis gas discharged from the crude gas purification unit 122.

On the other hand, when the concentration of the poisoning substance acquired from the concentration meter 124 is higher than a predetermined value, for example, higher than 100 volppm, the control unit 121 determines that the concentration of the poisoning substance input from the concentration meter 124 is high. In this case, control is performed to increase the branch flow rate of the synthesis gas supplied to the poisoning substance separation unit 125 among the synthesis gas discharged from the poisoning substance separation unit 126. When determining that the concentration of the poisoning substance is high, the control unit 121 performs control to increase the branch flow rate of the synthesis gas supplied to the poisoning substance separation unit 125 among the synthesis gas discharged from the crude gas purification unit 122.

As described above, the control unit 121 controls the branch valves 127 and 128 so that the concentration of the poisoning substance in the synthesis gas supplied to the FT synthesis reactor 20 becomes equal to or less than a predetermined value, for example, 100 volppm or less, based on the measurement value of the concentration of the poisoning substance in the synthesis gas input from the concentration meters 123 and 124. That is, the gas purifier 12 is configured to control the concentration of the poisoning substance in the synthesis gas supplied to the FT synthesis reactor 20 by providing a plurality of systems of poisoning substance removal lines for removing the poisoning substance from the synthesis gas whose concentration has been reduced to a predetermined level of the poisoning substance by the crude gas purification unit 122.

In the example of the gas purifier 12 described above, the poisoning substance separation units 125 and 126 are provided in two stages, but may be provided in three or more stages. In this case, it is possible to selectively control the number of stages of poisoning substance separation units to which the synthesis gas is supplied by providing a plurality of stages of the poisoning substance separation units in series along the flow direction of the synthesis gas and appropriately providing a branch valve on the inflow side. Thus, the concentration of the poisoning substance in the synthesis gas supplied to the FT synthesis reactor 20 can be adjusted. In addition, the branch valves 127 and 128 can be appropriately provided in the number of stages in which the poisoning substance separation units 125 and 126 are provided or the number of lines to be branched. With the configuration of the gas purifier 12 according to the first example, the concentration of the poisoning substance in the synthesis gas supplied to the FT synthesis reactor 20 can be controlled, thus suppressing deterioration in the catalyst capable of synthesizing a hydrocarbon from the synthesis gas containing carbon oxide.

### (Second Example)

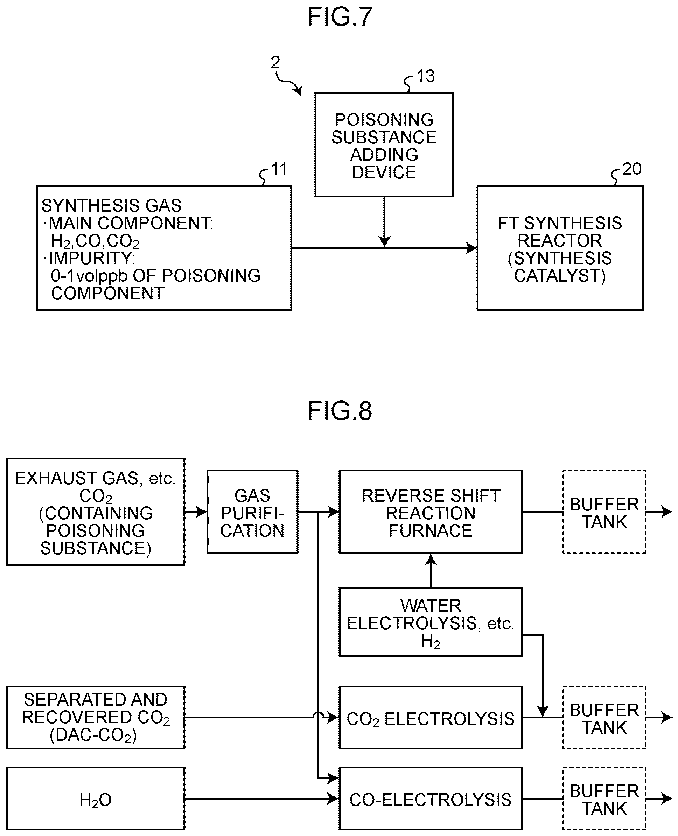

FIG. 7 is a schematic view for describing a method for supplying a synthesis gas according to the second example, and FIG. 8 is a schematic view illustrating a first specific example of a synthesis gas supply unit 11 according to the second example.

As illustrated in FIG. 7, the synthesis gas contains a main component and impurities, and the concentration of the poisoning substance as an impurity is an extremely small amount of about 0 volppb or more and 1 volppb or less. That is, in the second example, a case in which the synthesis gas contains almost no temporary poisoning substance or permanent poisoning substance as a poisoning substance is considered. The main component of the synthesis gas is the same as that in the first example.

In the second example, a synthesis gas containing almost no poisoning substance is supplied from the synthesis gas supply unit 11 to the FT synthesis reactor 20. The synthesis gas supply unit 11 is configured in the same manner as in the first embodiment. A poisoning substance adding device 13 as a poisoning substance addition unit is configured to add a poisoning substance to a reaction gas from a supply route of the reaction gas from the synthesis gas supply unit 11 to the FT synthesis reactor 20 and to adjust the concentration of the poisoning substance contained in the synthesis gas. The poisoning substance adding device 13 adds the poisoning substance to the supply route of the synthesis gas to contain the poisoning substance in the synthesis gas supplied to the FT synthesis reactor 20. The poisoning substance adding device 13 adds the poisoning substance to the synthesis gas so that the concentration of the poisoning substance is more than 0 volppm and 1 volppm or less, preferably 0.01 volppm or more and 0.1 volppm or less. In the second example, it is preferable to select one kind of poisoning substance as the poisoning substance for adding to the synthesis gas, but a plurality of kinds of poisoning substances may be added. When a plurality of poisoning substances are added, the concentration of the poisoning substances is preferably controlled by adjusting the addition amount based on the total concentration of the poisoning substances. In other words, it is preferable to adjust the addition amount so that the total concentration of each of the plurality of poisoning substances is more than 0 volppm and 1 volppm or less.

The synthesis gas to which the poisoning substance has been added is supplied to the FT synthesis reactor 20, which contains a synthesis catalyst, to generate a hydrocarbon through the FT method. In the second example, the synthesis gas supply unit 11 and the poisoning substance adding device 13 constitute a synthesis gas supply device 2 as a supply device.

As illustrated in FIG. 8, specifically, a method of separating and recovering exhaust gas from a power plant or the like or CO₂ in the atmosphere and converting the gas into CO gas by reverse shift reaction or electroreduction, followed by mixing with H₂ generated by water electrolysis or the like to supply the mixture, can be adopted as for the synthesis gas supply unit 11 including the poisoning substance according to the second example. In FIG. 8, the dotted line portion may or may not be installed in the synthesis gas supply unit 11.

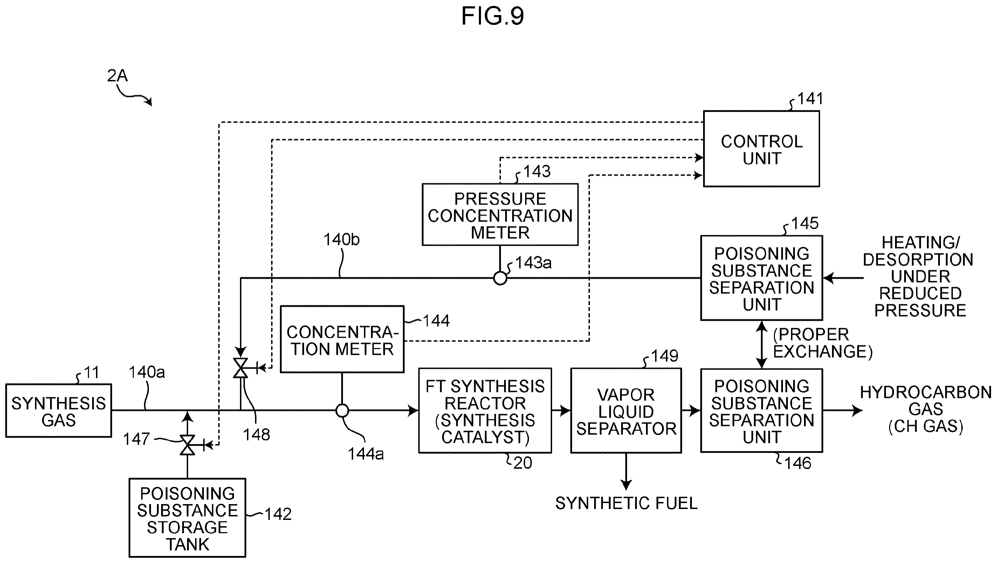

Next, a synthesis gas supply device capable of realizing the synthesis gas supply method according to the second embodiment will be described. FIG. 9 is a block diagram illustrating Example 2 of the synthesis gas supply device according to the second example.

As illustrated in FIG. 9, a synthesis gas supply device 2A according to Example 2 includes a control unit 141, a poisoning substance storage tank 142, a pressure concentration meter 143, a concentration meter 144, poisoning substance separation units 145 and 146, switching valves 147 and 148, and a vapor liquid separator 149. The control unit 141, the concentration meter 144, and the poisoning substance separation units 145 and 146 are configured similarly to the control unit 121, the concentration meter 124, the poisoning substance separation units 125 and 126, and the branch valves 127 and 128 in the first example described above, respectively. Both the poisoning substance separation units 145 and 146 are configured to be removable and mutually exchangeable and to discharge the poisoning substance by heating or decompression. That is, for example, when the adsorption amount or the storage amount of the poisoning substance in the poisoning substance separation unit 146 becomes a predetermined value or more, the poisoning substance separation unit 146 is appropriately exchanged with the poisoning substance separation unit 145. In this case, the poisoning substance separation unit 145 is in a state in which the poisoning substance is discharged to reduce the adsorption amount and the storage amount of the poisoning substance, that is, in a state after regeneration, and thus, can adsorb or store the poisoning substance after replacement.

The poisoning substance storage tank 142 is configured to store a poisoning substance such as ammonia (NH₃). The pressure concentration meter 143 includes a pressure concentration sensor 143a capable of measuring pressure and concentration. The pressure concentration sensor 143a is configured to measure the pressure of the synthesis gas and the concentration of the poisoning substance in a line 140b, which is a pipe joining a line 140a communicating the synthesis gas supply unit 11 and the FT synthesis reactor 20 from the release side of the poisoning substance separation unit 145 functioning as the poisoning substance release unit. The pressure concentration meter 143 outputs the measured pressure of the gas and the concentration of the poisoning substance to the control unit 141. The concentration meter 144 includes a concentration sensor 144a capable of measuring concentration. The concentration sensor 144a is configured to measure the concentration of the poisoning substance in the synthesis gas flowing in the line 140a supplied to the FT synthesis reactor 20. The concentration meter 144 outputs the measured concentration of the poisoning substance in the synthesis gas to the control unit 141.

Through the execution of the program, the control unit 141 controls each constituent unit such as the switching valves 147 and 148 based on the information on the pressure in the pipe and the concentration of the poisoning substance supplied from the pressure concentration meter 143 and the concentration meter 144 to exhibit a function that meets a predetermined purpose. Specifically, the switching valves 147 and 148 are controlled by the control unit 141 to open and close, adjust the flow rate of gas, and the like. The flow rate of the poisoning substance flowing from the poisoning substance storage tank 142 into the line 140a can be adjusted by the switching valve 147 controlled by the control unit 141. The flow rate of the poisoning substance discharged from the poisoning substance separation unit 145 and caused to flow into the line 140a can be adjusted by the switching valve 148 controlled by the control unit 141.

The vapor liquid separator 149 is provided at the subsequent stage of the FT synthesis reactor 20 along the flow direction of the synthesis gas. The vapor liquid separator 149 is configured to allow vapor-liquid separation for the gas after the FT synthesis is performed. The liquid component separated in the vapor liquid separator 149 is recovered as a synthetic fuel. The gas separated in the vapor liquid separator 149 is supplied to the poisoning substance separation unit 146. In the poisoning substance separation unit 146, the poisoning substance contained in the gas is separated and removed by activated carbon or the like, for example. The hydrocarbon gas (CH gas) after the poisoning substance is separated is used for, for example, combustion heating of a synthesis gas furnace, raw materials, power generation, or the like.

In the second example, the poisoning substance in the synthesis gas supplied from the synthesis gas supply unit 11 is not contained or is very small. Therefore, the synthesis gas supply device 2A performs a process of adding a necessary amount of the poisoning substance so that the concentration of the poisoning substance in the synthesis gas supplied to the FT synthesis reactor 20 becomes about a predetermined value, for example, about 0.1 volppm.

That is, the concentration meter 144 first measures the concentration of the poisoning substance in the synthesis gas flowing in the line 140a at a preceding stage of the FT synthesis reactor 20 in the synthesis gas supply device 2A. As a result, the concentration of the poisoning substance mixed from the poisoning substance storage tank 142 and the poisoning substance separation unit 145 is measured with respect to the synthesis gas containing almost no poisoning substance. A measurement value of the concentration with the concentration meter 144 is output to the control unit 141. Based on the acquired measurement value of the concentration of the poisoning substance, the control unit 141 controls the switching valves 147 and 148 so that the concentration of the poisoning substance of the poisoning substance such as NH₃ becomes a predetermined value to control the flow rate of the gas (poisoning substance-containing gas) containing the poisoning substance flowing into the line 140a from the poisoning substance storage tank 142 and the poisoning substance separation unit 145.

The product gas generated in the FT synthesis reactor 20 is supplied to the vapor liquid separator 149, and the liquid component is separated and recovered as a synthetic fuel. The product gas separated by the vapor liquid separator 149 is supplied to the poisoning substance separation unit 146. The product gas contains a poisoning substance which is not adsorbed on the catalyst provided in the FT synthesis reactor 20. The poisoning substance contained in the product gas is adsorbed on, for example, activated carbon in the poisoning substance separation unit 146 and is separated and recovered, and the hydrocarbon gas is recovered.

Through the process of separating and recovering the poisoning substance, the poisoning substance is adsorbed or stored on the activated carbon in the poisoning substance separation unit 146.. For example, when the adsorption amount of the adsorption tower constituting the poisoning substance separation unit 146 is saturated, the poisoning substance separation unit can be replaced with the poisoning substance separation unit 145 in which the adsorption amount or the storage amount of the poisoning substance is small, or can be replaced with a poisoning substance part separation unit including another adsorption tower or the like. For the poisoning substance separation units 145 and 146 in which the adsorption amount or the storage amount of the poisoning substance are large, a gas containing the poisoning substance is generated by performing a regeneration treatment such as heating or decompression, and introduced into the line 140a at a preceding stage of the FT synthesis reactor 20, allowing the poisoning substance to be reused and reducing the cost.

In addition, it is also possible to combine the first example and the second example described above. That is, it is also possible to adopt a configuration in which a synthesis gas supply unit for supplying a synthesis gas to the FT synthesis reactor 20 is configured using the gas purifier 12 according to the first example, and the synthesis gas supply device in the second example is provided at the subsequent stage. For example, when the synthesis gas contains a large amount of H₂S as a permanent poisoning substance and does not contain or contains a small amount of NH₃ as a temporary poisoning substance, H₂S may be removed at a preceding stage, and NH₃ may be added at a subsequent stage. As a result, the catalyst can be regenerated in the FT synthesis reactor 20, and therefore, the replacement interval of the catalyst can be greatly extended.

According to the embodiment described above, in the catalyst capable of synthesizing the hydrocarbon (CHₓ) from the synthesis gas containing the carbon oxide (COₓ), the rate of decline in reactivity of the FT synthesis catalyst can be reduced, thus suppressing the deterioration of the FT reaction catalyst.

In addition, further effects and modifications can be easily derived by those skilled in the art. The broader aspects of the present invention are not limited to the specific details and representative embodiments presented and described above. Accordingly, various changes may be made without departing from the spirit or scope of the general inventive concept as defined by the appended claims and their equivalents.

### Industrial Applicability

A method and device for supplying to a catalyst according to the present invention are suitably applied to a catalyst for producing a liquid fuel by reacting a mixed gas of carbon oxide and hydrogen.

### Reference Signs List

1, 2, 2A MIXED GAS SUPPLY DEVICE
11 SYNTHESIS GAS SUPPLY UNIT
12 GAS PURIFIER
13 POISONING SUBSTANCE ADDING DEVICE
20 FT SYNTHESIS REACTOR
121, 141 CONTROL UNIT
122 CRUDE GAS PURIFICATION UNIT
123, 124, 144 CONCENTRATION METER
123a, 124a, 144a CONCENTRATION SENSOR
125, 126, 145, 146 POISONING SUBSTANCE SEPARATION UNIT
127, 128 BRANCH VALVE
129 PUMP
140a, 140b LINE
142 POISONING SUBSTANCE STORAGE TANK
143 PRESSURE CONCENTRATION METER
143a PRESSURE CONCENTRATION SENSOR
147, 148 SWITCHING VALVE
149 VAPOR LIQUID SEPARATOR

## Claims

1. A method for supplying a synthesis gas containing hydrogen gas and carbon oxide gas to a catalyst capable of producing a hydrocarbon from the synthesis gas, the method comprising:
supplying the synthesis gas to the catalyst as a gas containing a poisoning substance that is poisonous to the catalyst.

2. The method for supplying to the catalyst according to claim 1, wherein
a concentration of the poisoning substance in the synthesis gas is more than 0 volppm and 1.0 volppm or less.

3. The method for supplying to the catalyst according to claim 1, wherein
a concentration of the poisoning substance in the synthesis gas is 0.01 volppm or more and 0.2 volppm or less.

4. The method for supplying to the catalyst according to claim 1, wherein
a concentration of the poisoning substance in the synthesis gas is 0.02 volppm or more and less than 0.1 volppm.

5. The method for supplying to the catalyst according to claim 1, wherein
the poisoning substance is a temporary poisoning substance having a temporary poisonous property capable of regenerating the catalyst.

6. The method for supplying to the catalyst according to claim 5, wherein
the temporary poisoning substance is at least one compound selected from the group consisting of ammonia (NH₃), hydrogen cyanide (HCN), phosphine (PH₃), sodium chloride (NaCl), and potassium chloride (KCl).

7. The method for supplying to the catalyst according to claim 1, wherein
the poisoning substance is a permanent poisoning substance having a permanent poisonous property incapable of regenerating the catalyst.

8. The method for supplying to the catalyst according to claim 7, wherein
the permanent poisoning substance is at least one compound selected from a compound containing sulfur (S) and hydrogen chloride (HCl).

9. The method for supplying to the catalyst according to claim 7 or 8, wherein
the permanent poisoning substance is at least one compound selected from the group consisting of hydrogen sulfide (H₂S), carbonyl sulfide (COS), hydrogen chloride (HCl), and arsine (AsH₃).

10. The method for supplying to the catalyst according to claim 1, wherein
the catalyst includes a metal-based catalyst that contains a metal compound having activity in a Fischer-Tropsch synthesis reaction and produces a hydrocarbon from the synthesis gas, and a carrier catalyst that contains a metal oxide supporting the metal-based catalyst.

11. The method for supplying to the catalyst according to claim 1, wherein
the catalyst includes a metal-based catalyst that contains a metal and a metal compound having activity in a Fischer-Tropsch synthesis reaction and produces a hydrocarbon from the synthesis gas, and a carrier catalyst that contains a zeolite supporting the metal-based catalyst, the metal and the metal compound including cobalt and at least one metal selected from the group consisting of manganese and ruthenium.

12. The method for supplying to the catalyst according to claim 1, comprising:
controlling, by a control unit including hardware, an amount of the poisoning substance introduced into the synthesis gas supplied to the catalyst to adjust a concentration of the poisoning substance contained in the synthesis gas supplied to the catalyst.

13. The method for supplying to the catalyst according to claim 12, wherein
before supplying the synthesis gas to the catalyst, the control unit branches at least a part of the synthesis gas from a line through which the synthesis gas is supplied to the catalyst, removes the poisoning substance at a preceding stage of the catalyst, and then supplies the synthesis gas to the catalyst together with a remainder of the synthesis gas.

14. The method for supplying to the catalyst according to claim 12, comprising:
recovering the poisoning substance on a discharge side of the catalyst; and mixing at least a part of the recovered poisoning substance into the synthesis gas supplied to the catalyst.

15. A supply device, comprising:
a synthesis gas supply unit configured to supply a synthesis gas containing hydrogen gas, carbon oxide gas, and a poisoning substance that is poisonous to a catalyst; and
a gas purification unit configured to adjust a concentration of the poisoning substance contained in the synthesis gas supplied from the synthesis gas supply unit, wherein
the supply device is configured to supply the synthesis gas to a synthesis reactor including the catalyst, the synthesis reactor being configured to synthesize a hydrocarbon from the hydrogen gas and the carbon oxide gas contained in the synthesis gas.

16. The supply device according to claim 15, wherein
the gas purification unit adjusts the concentration of the poisoning substance in the synthesis gas to be more than 0 volppm and 1.0 volppm or less.

17. The supply device according to claim 15, wherein
the gas purification unit includes at least one poisoning substance separation unit configured to separate the poisoning substance from the synthesis gas, and a control unit including hardware capable of controlling a flow rate of supplying the synthesis gas to the poisoning substance separation unit,
at a preceding stage of the poisoning substance separation unit, the synthesis gas is caused to flow into at least one of a first route in which the synthesis gas is supplied to the synthesis reactor without passing through the poisoning substance separation unit and a second route in which the synthesis gas is supplied to the synthesis reactor after being supplied to the poisoning substance separation unit, and
the control unit adjusts the concentration of the poisoning substance by controlling the flow rate of the synthesis gas flowing into the first route and the flow rate of the synthesis gas flowing into the second route.

18. The supply device according to claim 15, comprising:
a plurality of stages of the poisoning substance separation unit in series along a flow direction of the synthesis gas, wherein
the supply device is configured to adjust the concentration of the poisoning substance by selecting the number of stages of the poisoning substance separation unit to which the synthesis gas is supplied.

19. A supply device, comprising:
a synthesis gas supply unit configured to supply a synthesis gas containing hydrogen gas and carbon oxide gas; and
a poisoning substance addition unit capable of adding a poisoning substance that is poisonous to a catalyst to the synthesis gas supplied from the synthesis gas supply unit, wherein
the supply device is configured to supply the synthesis gas to a synthesis reactor including the catalyst, the synthesis reactor being configured to synthesize a hydrocarbon from the hydrogen gas and the carbon oxide gas contained in the synthesis gas.

20. The supply device according to claim 19, wherein
the poisoning substance addition unit adds the poisoning substance to the synthesis gas so that a concentration of the poisoning substance in the synthesis gas is more than 0 volppm and 1.0 volppm or less.

21. The supply device according to claim 19, comprising:
a plurality of poisoning substance separation units configured to recover and release the poisoning substance, wherein
on a downstream side of the synthesis reactor along a flow direction of the synthesis gas, some of the poisoning substance separation units among the plurality of poisoning substance separation units are configured to recover the poisoning substance, and on an upstream side of the synthesis reactor, the remaining poisoning substance separation units of the plurality of poisoning substance separation units are configured to introduce the poisoning substance into the synthesis gas.

22. The supply device according to claim 21, wherein
the some of the poisoning substance separation units and the remaining poisoning substance separation units are configured to be exchangeable.

23. The supply device according to claim 15 or 19, wherein
the catalyst includes a metal-based catalyst that contains a metal and a metal compound having activity in a Fischer-Tropsch synthesis reaction and produces a hydrocarbon from the synthesis gas, and a carrier catalyst that contains a zeolite supporting the metal-based catalyst, the metal and the metal compound including cobalt and at least one metal selected from the group consisting of manganese and ruthenium.
